# EUROPEAN PATENT SPECIFICATION

(11) **EP 1 954 225 B1**
(45) Date of publication and mention of the grant of the patent: **10.08.2011**
(21) Application number: 06812737.2
(22) Date of filing: 03.11.2006
(51) Int. Cl.: A61F 7/00

(54) **DEVICE FOR HEATING CLOTHS AND METHOD FOR APPLYING SUCH A DEVICE**
VORRICHTUNG ZUR ERWÄRMUNG VON KLEIDUNG UND VERFAHREN ZUR ANWENDUNG DIESER VORRICHTUNG
DISPOSITIF DE CHAUFFAGE DES TISSUS ET PROCEDE D'APPLICATION D'UN TEL DISPOSITIF

(30) Priority: 16.11.2005 NL 1030436
(43) Date of publication of application: 13.08.2008
(73) Proprietor: JOYinCARE Group B.V., 9699 PP Vriescheloo (NL)
(72) Inventor: HUIZINGA, Jozef, 9699 PP Vriescheloo (NL); KRIJNSEN, Wilhelmus, Johannes, Marinus, NL-8071 NS Nunspeet (NL)
(74) Representative: Langenhuijsen, Bastiaan Wilhelmus Herman
(86) International application number: PCT/NL2006/050278
(87) International publication number: WO 2007/058533

(56) References cited:
- EP-A- 1 084 670
- US-A- 4 419 568
- US-A- 5 004 894
- US-B1- 6 235 047
- US-B1- 6 316 750

## Description

The invention relates to a device for heating moist cloths. The invention also relates to a method for applying such a device.

Moist cloths, and in particular moist flannels, are used in care institutions for the care of patients. The flannels are here pre-moistened during the production process and provided with a packaging, whereby the flannels no longer have to be moistened manually, which considerably enhances convenience of use for care staff. For the comfort of the patient the moist flannels are heated. Heating of the flannels generally takes place by means of a microwave or by placing the (sealed) packaging in hot water. After heating of the flannels, they are transported to a patient requiring care. The drawback hereof is that the flannel cools considerably during transport, this at the expense of the comfort of the patient. In addition, a packaging is generally provided with a plurality of moist flannels, whereby the flannels which are not used first have generally already cooled to room temperature by the time they are removed from the packaging. Re-heating of an already opened package incorporating one or more flannels is generally undesirable from the viewpoint of hygiene, all the more so as flannels from a single package are generally used for a plurality of patients. Interim heating of the remaining flannels situated in the package would moreover result in a considerable increase in the daily walking distances of care staff, this also being undesirable. A device having the features of the preamble of claim 1 is known from US-B-6 316 750.

The invention has for its object to provide a device for heating moist cloths, using which the moist cloths can be kept warm for a relatively long period.

The invention provides for this purpose a device of the type stated in the preamble, comprising: at least one heating station comprising permanently connected heating means for heating at least one moist cloth provided with a packaging, and at least one cassette arranged releasably in the heating station and adapted to hold the at least one moist cloth provided with the packaging, wherein the cassette is provided with at least one accommodating space for accommodating at least a part of the heating means. Accommodating at least a part, and preferably a substantial part, of the heating means in the accommodating space of the cassette is particularly advantageous because the heat generated by the heating means can be transmitted, not via the cassette but in relatively efficient manner directly from a position inside the cassette, to the packaging and the at least one moist cloth received therein. The packaging is thus heated from the core of the cassette and therefore not via the cassette (from outside the cassette into the cassette), which is particularly advantageous from an energy viewpoint since the heat generated by the heating means can be employed with maximum efficiency for heating the packaging and the at least one moist cloth received therein. Because heating takes place from the core of the cassette close to the packaging, the heat losses can be kept to a minimum. Application of the device according to the invention thus has the significant advantage that the packaging can be heated relatively efficiently, wherein the heat stored in the moist cloth can be retained for a relatively long period of time. After heating of the one or more moist cloths the cassette can be taken out of the heating station and transported to a patient requiring care. The heated moist cloth can there be taken out of the cassette and used. Hardly any cooling of the moist cloths will occur during transport of the one or more moist cloths from the heating station to the patient because the heat stored in the cloths can be conserved by means of applying the cassette, particularly since the atmosphere present in the cassette and surrounding the packaging has a heat-insulating effect. Because the moist cloths can be held for a relatively long time at or at least close to the temperature of use, (considerable) premature cooling of the cloths can be prevented, which considerably increases the comfort of the patient. The cassette is preferably person-specific, in particular patient-specific, whereby unused moist cloths can remain in the cassette and can in this way be stored in relatively hygienic manner and be reheated later. Mixing of pathogenic bacteria and viruses originating from different patients can in this way be prevented. Each packaging can be provided with a single moist cloth, but is generally provided with a plurality of, usually eight, moist cloths. The moist flannels can be of diverse types, but are preferably formed by flannels, in particular disposable flannels, which facilitates care of patients. The cloths are pre-moistened by means of water or any other liquid, to which skin-cleansing and/or skin-caring additives are optionally added. The cassette can be adapted to hold a single packaging, but it is also possible to envisage the cassette being adapted to hold a plurality of packages simultaneously. It is also possible to envisage the packaging for the at least one moist cloth forming an (integral) part of the cassette and in fact being formed by the cassette, whereby the at least one moist cloth is arranged in the cassette in a situation where it is not pre-packed in a separate (foil) packaging.

In order to enable the moist cloths to be kept for a longer time at a temperature which is comfortable for a patient, it is advantageous if the heat-insulating capacity of the cassette is maximized. The cassette is therefore preferably manufactured at least partly from a heat-insulating material, whereby the temperature of the moist cloths can be retained for longer. It is possible here to envisage the cassette being for instance provided with a lining manufactured from glass wool and/or rock wool. In another preferred embodiment the cassette is provided with heat-accumulating means. The heat-accumulating means are adapted to also be heated in the heating station, wherein the heat can be retained for a relatively long time. The heat-accumulating means are preferably manufactured from a material with a relatively high heat capacity, more preferably a gel. A particular advantage of a gel relative to a liquid is that gel is relatively viscous, whereby gel will not flow, or is at least less likely to do so, when the cassette is tilted. The specific heat capacity per unit of volume of a gel will usually also be (substantially) greater than the specific heat capacity per unit of volume of a solid, since solid insulating materials are usually given a partially porous form, while the gel forms a substantially homogeneous insulating mass, whereby use of a gel will generally also be preferred to use of a solid.

The heat-accumulating means can form part of a housing of the cassette, although it is also possible to envisage these means being accommodated in a separate housing in the cassette, close to the moist cloths. It will be apparent that means other than gel can also be applied in order to allow realization of the desired heat accumulation.

In a preferred embodiment the cassette is provided with a closable first passage opening to enable placing in the cassette of at least one moist cloth provided with a packaging, and with a closable second passage opening to enable removal of the at least one moist cloth from the cassette. The first passage opening will generally be given significantly greater dimensions than the second passage opening in order on the one hand to be able to facilitate positioning of one or more packages in the cassette, and on the other to be able to optimize the removal of one or more cloths in relatively efficient manner, wherein the heat loss which occurs when the second passage opening is opened can be minimized. In a particular preferred embodiment the cassette comprises a base structure and a top structure co-acting with the base structure, wherein the base structure and the top structure co-act with each other as shell parts. The first passage opening is opened by removing the top structure, whereafter a packaging can be placed in the cassette or removed from the cassette. The top structure is preferably provided with the second passage opening for enabling removal of the moist cloths, this second passage opening being closable by means of a closing element, preferably a flap-like element. The base structure and the top structure are preferably connected to each other in mutually pivotable manner so as to be able to facilitate filling of the cassette with optionally pre-moistened cloths. In a particular preferred embodiment the base structure and the top structure are mutually releasable and optionally connected pivotally to each other. Being able to release the base structure and the top structure from each other has the important advantage that the cassette can be cleaned relatively efficiently, for instance in a dishwasher, which generally enhances the hygiene of the cassette substantially. This releasable coupling of the base structure and the top structure moreover facilitates the production process as well as the storage and transport of a quantity of manufactured cassette(s) to a user of the device according to the invention.

The heating means can be of very diverse nature, although the heating means preferably comprise at least one heating element. The heating element is adapted to generate direct (sensible) heat, generally by generating infrared radiation, whereby the whole packaging, the at least one cloth received therein, and optionally the cassette, and the heat-accumulating means optionally forming part of the cassette can be heated in relatively intensive and complete manner. The heating element will preferably be formed by a resistive heating track. The heating track is herein preferably accommodated in a substrate, more preferably formed by a plate which does not conduct electric current. The substrate is preferably formed here by a mat formed from rubber or foil. So as to be able to prevent overheating of the heating element, and thereby of the packaging and the at least one moist cloth received therein, the heating element is coupled to an NTC resistor. Through use of a specific NTC resistor it is possible to switch off the heating element when a predetermined temperature is reached, preferably lying at or just above the temperature of use of the moist cloths. The heating element is more preferably coupled to a thermostat. It has been found that a thermostat functions (considerably) more precisely than an NTC resistor, whereby application of a thermostat is recommended to enable timely switch-off of the heating element.

The base structure of the cassette, in particular a bottom element forming part of the base structure, will be provided with the at least one accommodating space for at least a part of the heating means in order to enable optimization of the heat transfer from the heating element to the packaging and the one or more moist cloths received therein.

In another preferred embodiment of the device according to the invention the heating means are (also) adapted to generate electromagnetic radiation for (indirect) heating of the at least one moist cloth. The electromagnetic radiation is preferably formed by microwaves with a wavelength of substantially between 0.1 millimetre and 1 cm. It is however also possible to envisage heating the packaging by means of infrared radiation, as already indicated in the foregoing. Heating by making use of infrared radiation is generally less harmful to people than microwaves, and is generally preferred due to a more effective heat transfer.

The device preferably comprises a supply container for a plurality of packages, wherein each package is provided with at least one moist cloth. The device according to the invention thus acquires an additional functionality of storing packages still to be used in relatively efficient, hygienic and practical manner, wherein each package is provided with one or more moist cloths. The supply container is herein preferably coupled to the heating station in order to limit the mutual distance between the supply container and the heating station.

As stated above, the applied cassette is person-specific, in particular patient-specific. Since a plurality of patients must generally be cared for in a care institution, the device preferably comprises a plurality of (person-specific) cassettes. In order to increase the heating capacity of the device, the device preferably comprises a plurality of heating stations, whereby a plurality of cassettes can be heated simultaneously.

In a preferred embodiment the at least one cassette comprises sensor means for identifying the at least one moist cloth received in the cassette. Using the sensor means the cassette can determine whether or not a packaging is situated in the cassette and/or whether a moist cloth is situated in the cassette. In addition to the presence registration which can be carried out using the sensor means, it is also possible to envisage the sensor means being adapted to detect the nature of the packaging and/or of the moist cloth. In this manner it is for instance possible to determine the type of cloth, and optionally the number of cloths, situated in the cassette. The sensor means (or detection means) forming part of the cassette can for instance identify the packaging and/or the at least one cloth here on the basis of external features such as for instance colour or shine. It is however also possible to envisage each packaging and/or each cloth being provided with a chip carrying information, wherein the information stored on the chip can be read by the sensor means. The information recorded by the detection means will usually be processed by a central processing unit such as for instance a computer, and/or the detected information can be shown for passers-by via a display, whereby information about the nature and/or the number of cloths present in the cassette(s) is provided to these passers-by, usually care staff.

The invention also relates to an assembly of a device according to the invention and at least one moist cloth provided with a packaging. A plurality of moist cloths are preferably received in each packaging. The moist cloths are preferably formed here by flannels. Each flannel herein forms a (woven) washcloth which a carer can arrange round his or her hand so as to then be able to care for a patient.

The invention also relates to a cassette for use in a device according to the invention. The cassettes can be marketed separately.

The invention furthermore relates to a method for applying a device according to the invention, comprising the steps of: A) positioning at least one moist cloth provided with a packaging in the cassette, B) placing the cassette in the heating station, wherein the heating means are at least partly accommodated in the accommodating space arranged in the cassette, C) allowing the at least one moist cloth to be heated by the heating means, D) removing the cassette from the heating station, and E) removing at least one heated moist cloth from the cassette. Advantages of the method according to the invention have already been discussed at length in the foregoing. The moist cloth is preferably formed by a flannel.

The invention will be elucidated on the basis of non-limitative exemplary embodiments shown in the following figures. Herein:
figure 1 shows a schematic view of a device according to the invention,
figure 2 shows a perspective view of the device according to figure 1,
figure 3 shows a perspective view of another device according to the invention,
figure 4a shows a perspective view of an assembly of a cassette as used in the device according to figures 2 and 3, and a packaging in which moist flannels are received,
figure 4b shows a perspective view of an assembly of an alternative cassette and a packaging in which moist flannels are received,
figure 4c shows a perspective view of yet another cassette for use in a device according to the invention, and
figure 5 shows a schematic cross-section of a heating station and a cassette arranged in the heating station.

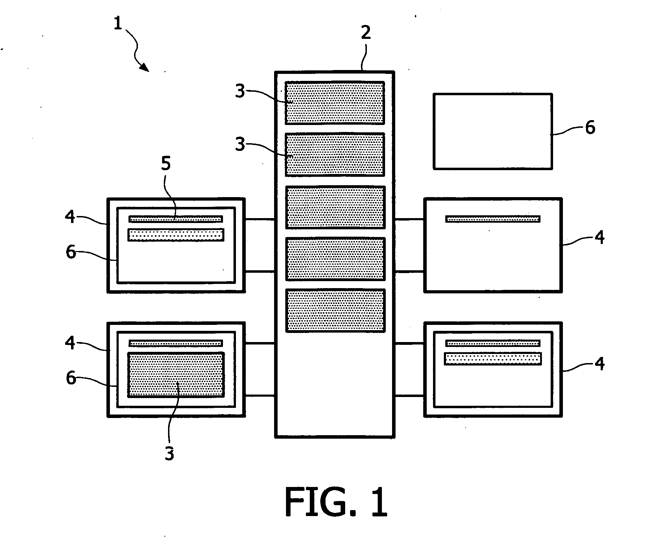

Figure 1 shows a schematic view of a device 1 according to the invention. Device 1 comprises a supply container 2 for a plurality of packages 3, wherein a plurality of moist flannels, preferably eight, are received in each packaging. Care staff can care for patients using the flannels. In order to increase the comfort for the patient the flannels must be heated. For this purpose the device comprises a plurality of heating stations 4 coupled to the supply container and adapted to enable heating of the flannels received in the packages. Each heating station 4 comprises for this purpose a heating element 5, such as for instance a heating mat provided with a heating track. During use a packaging 3 (filled with flannels) is arranged in a heat-insulating cassette 6, whereafter cassette 6 and the packaging 3 received therein are positioned in heating station 4. Cassette 6 is herein provided with an accommodating space (not shown) for at least a part of heating element 5, so that the heating element makes contact with or is at least positioned close to packaging 3 so as to be able to optimize the heat transfer from the heating element to packaging 3, and thereby to the flannels received therein. After reaching a desired temperature of the heating element, of the atmosphere in cassette 6, and/or of packaging 3, or at least the flannels received therein, heat-insulating cassette 6 is removed from heating station 4 by the carer and transported to a patient requiring care. Cassette 6 can then be opened close to the patient and one or more heated moist flannels can be removed from cassette 6 so as to enable care of a patient. Because cassette 6 has relatively good heat-insulating properties, the flannels can be kept at or just above a predetermined temperature of use for a relatively long time, whereby the comfort of the patient can be ensured. Cassette 6 preferably comprises for this purpose a heat-insulating housing and more preferably heat storage means so as to be able to retain part of the heat generated by heating element 5 for a (relatively long) period of time. The heat-insulating housing can for instance be at least partly provided here with glass wool and/or rock wool. The heat storage means can for instance be formed by a gel which is arranged in cassette 6, for instance in an accommodating space forming part of the housing of cassette 6 or in a separate housing. Cassette 6 is preferably patient-specific, which enhances the hygiene for the patients, the more so in that mixing of pathogens carried by different patients can in this way be avoided. In the shown exemplary embodiment device 1 is therefore a device for enabling care for four patients with the moist flannels, since four cassettes 6 are applied. It will however be apparent that numerous variations hereof are possible.

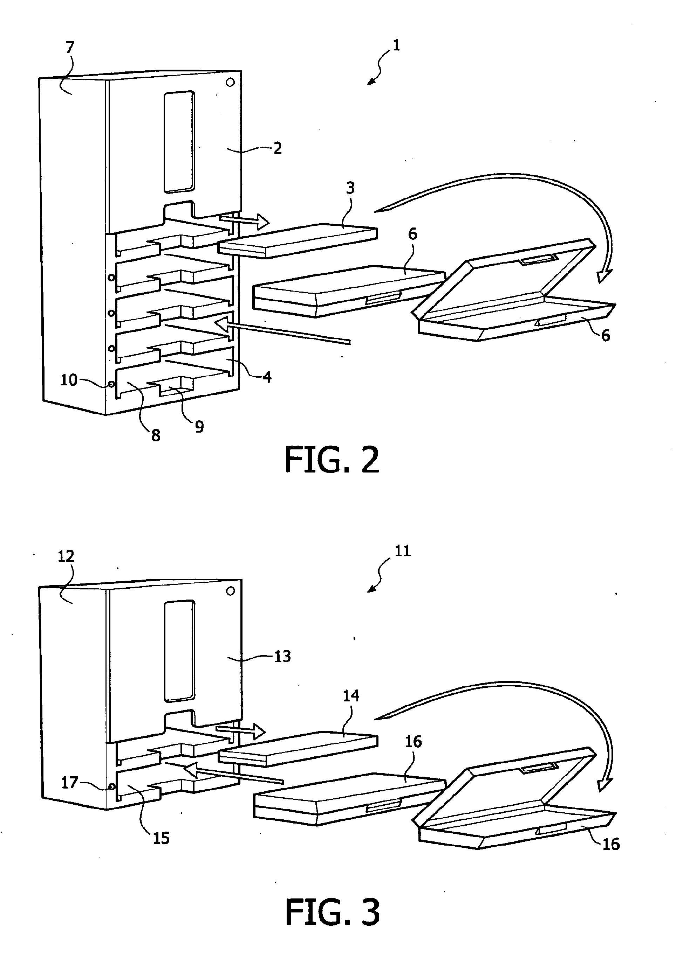

Figure 2 shows a perspective view of the device 1 according to figure 1. Shown clearly here is that supply container 2 and the four heating stations 4 are accommodated in a central housing 7, whereby the device takes a relatively compact form. Device 1 can be given a modular form, which means that the number of heating stations 4 to be applied, and thereby the number of cassettes 6 to be applied, can be modified relatively easily. Each heating station 4 is in fact formed by a slot 8 in which a cassette 6 can be placed. Removal of cassettes 6 from heating stations 4 can be facilitated by means of recesses 9 in housing 7. Along each heating station 4 is arranged an indicator lamp 10 to enable visualization of the situation in which packaging 3 and the flannels received therein have been sufficiently heated. During use of device 1 a packaging 3 will first be removed from supply container 2, whereafter the packaging is arranged in (opened) cassette 6. After closing of cassette 6 the cassette will be arranged in one of the heating stations 4, whereafter packaging 3 and the flannels received therein can be heated.

Figure 3 shows a perspective view of another device 11 according to the invention. Structurally, the device 11 corresponds substantially with device 1 shown in figure 2. Device 11 comprises a central housing 12 in which a supply container 13 for packages 14 of moist flannels is received. A single heating station 15 is also accommodated in central housing 12. By placing a packaging 14 in a heat-insulating cassette 16 and by further placing the filled cassette in heating station 15, packaging 14 and the flannels received therein can be heated to or just above a temperature of use that is comfortable for a patient. For this purpose cassettes 16 will be provided with an accommodating space for accommodating a directly or indirectly acting heating element enabling efficient heating of packages 14 and the flannels received therein. After reaching the temperature of use, an indicator lamp 17 will indicate that packaging 14 is ready for use, whereafter cassette 16 can be taken out of heating station 15 and transported as such to the patient, for removal there of the heated flannels from the cassette so as to be able to minimize heat losses during transport.

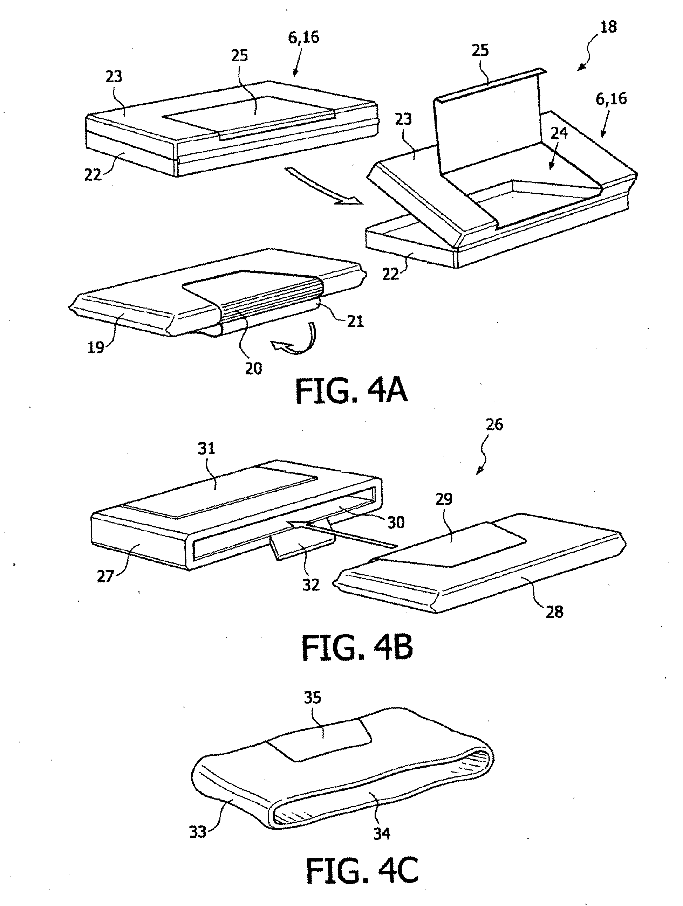

Figure 4a shows a perspective view of an assembly 18 of a cassette 6, 16 as used in device 1, 11 according to figures 2 and 3, and a packaging 19 in which moist flannels 20 are received. Packaging 19 is manufactured from a substantially medium-tight foil and is provided with a sealing strip 21 to enable sealing of the packaging so as to prevent flannels 20 drying out. Cassette 6, 16 comprises a substantially rigid base structure 22 and a substantially rigid top structure 23 connected pivotally to the base structure. Top structure 23 is provided with a passage opening 24 for flannels 20 taken out of packaging 19, this passage opening 24 being closable by means of a flap-like element 25 connected to top structure 23.

Figure 4b shows a perspective view of an assembly 26 of an alternative cassette 27 and a packaging 28 in which moist flannels are received. The substantially flexible packaging 28 is manufactured from a plastic foil and provided with a sealing element 29. The substantially rigid cassette 27, preferably manufactured from a relatively heat-insulating plastic, is provided with a passage opening 30 to enable sliding of packaging 28 into cassette 27 (see arrow). Cassette 27 is provided with a closing element 31, wherein the two closing elements 29, 31 lie substantially in line in the assembled situation of assembly 26. In order to facilitate placing of cassette 27 in a heating station and removal thereof from a heating station, cassette 27 is provided with a handle 32. An inner side of cassette 27 is preferably covered with a gel-like structure (not shown) with a relatively high heat capacity in order to improve the ability to keep the flannels warm for a longer time in cassette 27. Heating takes place by at least temporarily positioning at least a part of a heating element in cassette 27.

Figure 4c shows a perspective view of yet another cassette 33 for use in a device according to the invention. Cassette 33 is provided with a passage opening 34 to allow a packaging of optionally moist flannels to be received in cassette 33. Cassette 33 is also provided with a closing flap 35 to enable or prevent removal of flannels from cassette 33. In addition, cassette 33 is provided with an accommodating space (not shown) for a heating element to enable efficient heating of the packaging received in the cassette. The shown cassette 33 is manufactured from a flexible material. Cassette 33 is preferably manufactured from a closed foam structure, more preferably from neoprene, so as to be able to provide cassette 33 with a relatively high heat capacity.

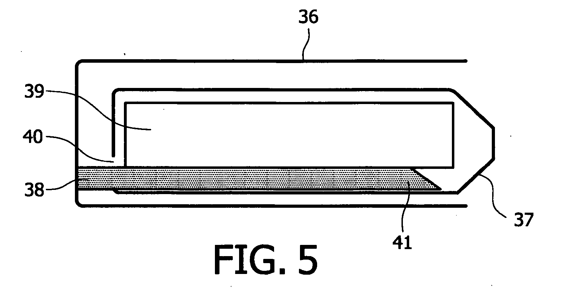

Figure 5 shows a schematic cross-section of a heating station 36 and a cassette 37 accommodated in heating station 36. Heating station 36 comprises a heating element 38 which, in the shown exemplary embodiment, is in direct contact with a packaging 39 for moist cloths to be heated received in cassette 37. Cassette 37 is provided for this purpose with a passage opening 40 for passage of heating element 38, whereby in the shown situation heating element 38 is positioned substantially inside cassette 37, whereby the heat transfer from heating element 38 to packaging 39 can be optimized. Heating element 37 has a substantially plate-like form, wherein a free outer end 41 of heating element 38 is substantially tapering in order to be able to facilitate slight lifting of packaging 39 in cassette 37 during sliding of cassette 37 into heating station 36.

It will be apparent that the invention is not limited to the exemplary embodiments shown and described here, but that numerous variants, which will be self-evident to the skilled person in this field, are possible within the scope of the appended claims.

## Claims

1. Device (1) for heating moist cloths, comprising:
- at least one heating station (4) comprising a permanently connected heating means (5) for heating at least one moist cloth provided with a packaging (3) and
- at least one cassette (6) arranged releasably in the heating station and adapted to hold the at least one moist cloth provided with the packaging,
**characterized in that** the cassette is provided with at least one accommodating space for accommodating at least a part of the heating means.

2. Device as claimed in claim 1, **characterized in that** the cassette is manufactured at least partly from a heat-insulating material.

3. Device as claimed in claim 1 or 2, **characterized in that** the cassette is provided with heat-accumulating means.

4. Device as claimed in claim 3, **characterized in that** the heat-accumulating means comprise gel.

5. Device as claimed in any of the foregoing claims, **characterized in that** the cassette is provided with a closable first passage opening to enable placing in the cassette of at least one moist cloth provided with a packaging, and with a closable second passage opening to enable removal of the at least one moist cloth from the cassette.

6. Device as claimed in any of the foregoing claims, **characterized in that** the heating means comprise at least one heating element.

7. Device as claimed in claim 6, **characterized in that** the heating element is coupled to a thermostat.

8. Device as claimed in any of the foregoing claims, **characterized in that** the heating means are adapted to generate electromagnetic radiation for heating the at least one moist cloth.

9. Device as claimed in any of the foregoing claims, **characterized in that** the device comprises a supply container for a plurality of packages, wherein each package is provided with at least one moist cloth.

10. Device as claimed in any of the foregoing claims, **characterized in that** the device comprises a plurality of heating stations and a plurality of cassettes.

11. Device as claimed in any of the foregoing claims, **characterized in that** the cassette comprises sensor means for identifying the at least one moist cloth received in the cassette.

12. Assembly of a device as claimed in any of the foregoing claims and at least one moist cloth provided with a packaging.

13. Assembly as claimed in claim 12, **characterized in that** a plurality of moist cloths are received in the packaging.

14. Assembly as claimed in claim 12 or 13, **characterized in that** the at least one moist cloths is formed by a flannel.

15. Cassette as claimed in any of claims 1-11, and for use in a device as claimed in any of the claims 1-11.

16. Method for applying a device as claimed in any of the claims 1-11, comprising the steps of:
A) positioning at least one moist cloth provided with a packaging in the cassette,
B) placing the cassette in the heating station, wherein the heating means are at least partly accommodated in the accommodating space arranged in the cassette,
C) allowing the at least one moist cloth to be heated by the heating means,
D) removing the cassette from the heating station, and
E) removing at least one heated moist cloth from the cassette.

17. Method as claimed in claim 16, **characterized in that** the moist cloth is formed by a flannel.

## Patentansprüche

1. Vorrichtung (1) zum Erhitzen von feuchten Tüchern, die Folgendes umfasst:
- mindestens eine Heizstation (4) mit ständig angeschlossenen Heizmitteln (5) zum Erhitzen von mindestens einem feuchten Tuch in einer Verpackung (3) und
- mindestens eine Kassette (6), die herausnehmbar in der Heizstation angeordnet und angepasst ist, um mindestens ein feuchtes Tuch in der Verpackung aufzunehmen, **dadurch gekennzeichnet, dass** die Kassette über mindestens einen Aufnahmeraum zur Aufnahme von mindestens einem Teil des Heizmittels verfügt.

2. Vorrichtung nach Anspruch 1, **dadurch gekennzeichnet, dass** die Kassette mindestens teilweise aus einem wärmeisolierenden Material hergestellt ist.

3. Vorrichtung nach Anspruch 1 oder 2, **dadurch gekennzeichnet, dass** die Kassette über wärmespeichernde Mittel verfügt.

4. Vorrichtung nach Anspruch 3, **dadurch gekennzeichnet, dass** die wärmespeichernden Mittel Gel umfassen.

5. Vorrichtung nach einem der oben genannten Ansprüche, **dadurch gekennzeichnet, dass** die Kassette mit einer ersten verschließbaren Durchgangsöffnung versehen ist, um mindestens ein feuchtes Tuch mit Verpackung in die Kassette legen zu können, sowie mit einer verschließbaren zweiten Durchgangsöffnung, um mindestens ein feuchtes Tuch aus der Kassette herausnehmen zu können.

6. Vorrichtung nach einem der oben genannten Ansprüche, **dadurch gekennzeichnet, dass** das Heizmittel mindestens ein Heizelement umfasst.

7. Vorrichtung nach Anspruch 6, **dadurch gekennzeichnet, dass** das Heizmittel mit einem Thermostat verbunden ist.

8. Vorrichtung nach einem der oben genannten Ansprüche, **dadurch gekennzeichnet, dass** die Heizmittel angepasst sind, um elektromagnetische Strahlung zum Erhitzen mindestens eines feuchten Tuchs zu erzeugen.

9. Vorrichtung nach einem der oben genannten Ansprüche, **dadurch gekennzeichnet, dass** die Vorrichtung einen Vorratsbehälter für eine Vielzahl von Verpackungen umfasst, wobei jede Verpackung mindestens ein feuchtes Tuch enthält.

10. Vorrichtung nach einem der oben genannten Ansprüche, **dadurch gekennzeichnet, dass** die Vorrichtung eine Vielzahl von Heizstationen und eine Vielzahl von Kassetten umfasst.

11. Vorrichtung nach einem der oben genannten Ansprüche, **dadurch gekennzeichnet, dass** die Kassette Sensormittel zur Erkennung mindestens eines feuchten Tuchs umfasst, das in der Kassette aufgenommen wurde.

12. Baugruppe einer Vorrichtung nach einem der oben genannten Ansprüche und mindestens ein feuchtes Tuch mit einer Verpackung.

13. Baugruppe nach Anspruch 12, **dadurch gekennzeichnet, dass** eine Vielzahl von feuchten Tüchern in der Verpackung aufgenommen wird.

14. Baugruppe nach Anspruch 12 oder 13, **dadurch gekennzeichnet, dass** mindestens ein feuchtes Tuch aus einem Lappen besteht.

15. Kassette nach einem der Ansprüche 1-11 zur Verwendung in einer Vorrichtung nach einem der Ansprüche 1-11.

16. Verfahren zur Anwendung einer Vorrichtung nach einem der Ansprüche 1-11, das folgende Schritte umfasst:
A) das Hineinlegen von mindestens einem feuchten Tuch mit Verpackung in eine Kassette,
B) das Ablegen der Kassette in der Heizstation, wobei die Heizmittel mindestens teilweise in dem in der Kassette angeordneten Aufnahmeraum untergebracht sind,
C) die Ermöglichung der Erhitzung von mindestens einem feuchten Tuch durch die Heizmittel,
D) das Entfernen der Kassette aus der Heizstation und
E) das Herausnehmen von mindestens einem erwärmten feuchten Tuch aus der Kassette.

17. Verfahren nach Anspruch 16, **dadurch gekennzeichnet, dass** das feuchte Tuch aus einem Lappen besteht.

## Revendications

1. Dispositif (1) pour chauffer des linges humides, comprenant :
- au moins un poste de chauffage (4) comprenant un moyen de chauffage (5) branché en permanence pour chauffer au moins un linge humide pourvu d'une enveloppe (3), et
- au moins une cassette (6) agencée amovible dans le poste de chauffage et adaptée pour contenir le au moins un linge humide pourvu d'une enveloppe,
**caractérisé en ce que** la cassette est pourvue d'au moins un espace de réception pour recevoir au moins une partie du moyen de chauffage.

2. Dispositif selon la revendication 1, **caractérisé en ce que** la cassette est fabriquée au moins en partie en un matériau isolant thermique.

3. Dispositif selon la revendication 1 ou 2, **caractérisé en ce que** la cassette est pourvue de moyens accumulateurs de chaleur.

4. Dispositif selon la revendication 3, **caractérisé en ce que** les moyens accumulateurs de chaleur comprennent du gel.

5. Dispositif selon l'une quelconque des revendications précédentes, **caractérisé en ce que** la cassette est pourvue d'une première ouverture de passage fermante pour permettre de placer dans la cassette au moins un linge humide pourvu d'une enveloppe, et d'une seconde ouverture de passage fermante pour permettre de retirer le au moins un linge humide de la cassette.

6. Dispositif selon l'une quelconque des revendications précédentes, **caractérisé en ce que** les moyens de chauffage comprennent au moins un élément chauffant.

7. Dispositif selon la revendication 6, **caractérisé en ce que** l'élément chauffant est couplé à un thermostat.

8. Dispositif selon l'une quelconque des revendications précédentes, **caractérisé en ce que** les moyens de chauffage sont adaptés pour produire un rayonnement électromagnétique pour chauffer le au moins un linge humide.

9. Dispositif selon l'une quelconque des revendications précédentes, **caractérisé en ce que** le dispositif comprend un récipient d'alimentation pour une pluralité d'enveloppes, étant entendu que chaque enveloppe est pourvue d'au moins un linge humide.

10. Dispositif selon l'une quelconque des revendications précédentes, **caractérisé en ce que** le dispositif comprend une pluralité de postes de chauffage et une pluralité de cassettes.

11. Dispositif selon l'une quelconque des revendications précédentes, **caractérisé en ce que** la cassette comprend des moyens de détection pour identifier le au moins un linge humide reçu dans la cassette.

12. Ensemble consistant en un dispositif selon l'une quelconque des revendications précédentes et en au moins un linge humide pourvu d'une enveloppe.

13. Ensemble selon la revendication 12, **caractérisé en ce qu'**une pluralité de linges humides sont reçus dans l'enveloppe.

14. Ensemble selon la revendication 12 ou 13, **caractérisé en ce que** le au moins un linge humide est fait en une flanelle.

15. Cassette selon l'une quelconque des revendications 1-11, et à utiliser dans un dispositif selon l'une quelconque des revendications 1-11.

16. Procédé pour appliquer un dispositif selon l'une quelconque des revendications 1-11, comprenant les étapes consistant :
A) à positionner au moins un linge humide pourvu d'une enveloppe dans la cassette ;
B) à placer la cassette dans le poste de chauffage, étant entendu que les moyens de chauffage sont au moins en partie reçus dans l'espace de réception aménagé dans la cassette ;
C) à permettre au au moins un linge humide d'être chauffé par les moyens de chauffage ;
D) à retirer la cassette du poste de chauffage, et
E) à retirer de la cassette au moins un linge humide chauffé.

17. Procédé selon la revendication 16, **caractérisé en ce que** le linge humide est fait en une flanelle.
